Europäisches Patentamt

European Patent Office

Office européen des brevets

(11) Publication number: **0 098 592**
**A2**

## EUROPEAN PATENT APPLICATION

(21) Application number: **83106571.9**

(51) Int. Cl.³: **A 61 M 5/00**

(22) Date of filing: **05.07.83**

(30) Priority: **06.07.82 JP 118114/82**
**19.07.82 JP 126504/82**
**19.07.82 JP 126505/82**

(43) Date of publication of application: **18.01.84**
**Bulletin 84/3**

(84) Designated Contracting States: **BE CH DE FR GB IT LI NL SE**

(71) Applicant: **Fujisawa Pharmaceutical Co., Ltd., 3, Doshomachi 4-chome Higashi-ku, Osaka-shi, Osaka 541 (JP)**

(72) Inventor: **Shichiri, Motoaki, 4-12-4, Komagawa, Higashisumiyoshi-ku Osaka-shi (JP)**
Inventor: **Hattori, Kiyoshi, No. 3-3-17, Sakasedai, Takarazuka-shi (JP)**
Inventor: **Nishiyama, Taichiro, No. 9-7-6, Higashinakahama, Joto-ku Osaka-shi (JP)**
Inventor: **Singuh, Yasuhiko, No. 2-6-31, Hattoriyutakamachi, Toyonaka-shi (JP)**
Inventor: **Takagi, Kiyotaka, No. 100-3, Katsurahisakatacho, Nishikyo-ku Kyoto-shi (JP)**
Inventor: **Higuchi, Mamoru, 6-6-13, Furuedai, Suita-shi (JP)**
Inventor: **Okamoto, Norihiko, No. 2-2-10, Midorigaoka, Ikeda-shi (JP)**
Inventor: **Okudaira, Toshiyuki, No. 1-7-15, Tagawakita, Yodogawa-ku Osaka-shi (JP)**

(74) Representative: **Tiedtke, Harro, Dipl.-Ing. et al, Patentanwaltsbüro Tiedtke-Bühling-Kinne-Grupe-Pellmann-Grams-Struif Bavariaring 4, D-8000 München 2 (DE)**

(54) **Portable artificial pancreas.**

(57) A portable artificial pancreas comprising a blood sugar detecting unit including electrode means to be inserted into the living body for measuring the blood sugar of the living body, an injection unit including a container for a blood sugar control agent and a feed pump for the control agent and adapted to inject the control agent into the living body, and an arithmetic control unit including a micorcomputer for calculating the blood sugar value from an output signal from the detecting unit and the dose of the control agent based on the value and controlling the pump of the injection unit in accordance with the result of calculation. An assembly including the blood sugar detecting unit and an assembly including the injection unit and the arithmetic cont rol unit are separate from each other and are individually attached to the body. The output signal of the detecting unit is transmitted to the control unit by radio.

## PORTABLE ARTIFICIAL PANCREAS

The present invention relates to a portable artificial pancreas, and more particularly to a portable artificial pancreas having the function of measuring blood sugar values (blood glucose concentrations) of the living body and injecting blood sugar control agents into the living body in accordance with the measurements.

Blood sugar control agents include hypoglycemic agents and hyperglycemic agents. Examples of hypoglycemic agents are insulin, etc., while exemplary of hyperglycemic agents are glucagon, glucose, etc.

Such artificial pancreas heretofore known include those of the so-called bedside type which comprise a blood sugar detecting unit, an injection unit having an insulin container, a glucagon container, an insulin feed pump and a glucagon feed pump and adpated to inject insulin or glucagon into the living body, an arithmetic control unit for calculating the blood sugar value from an output signal from the detecting unit and the dose of insulin or glucagon based on the value and controlling the pump concerned of the injection unit in accordance with the result of calculation, and a casing housing these units. However, the conventional

2                    0098592

artificial pancreas of the bedside type has the drawback
of being large-sized and limited to bedside use because
the blood sugar measuring electrode assembly (sensor) of
the detecting unit, the feed pumps of the injection unit
and the arithmetic control unit are relatively large.
Further because the blood sugar detecting unit is adapted
to determine blood sugar with use of blood collected from
the patient and diluted, the collection of blood heavily
burdens the patient, so that it is impossible to use the
device continuously for a long period of time.

An object of the present invention is to provide
a compact portable artificial pancreas.

Another object of the present invention is to
eliminate the need to collect the blood for the dertermina-
tion of blood sugar and to provide a portable artificial
pancreas which is usable continuously for a prolonged
period of time.

The present invention provides a portable
artificial pancreas which comprises a blood sugar detecting
unit including blood sugar measuring electrode means to be
inserted into the living body for measuring the blood sugar
of the living body, an injection unit including a container
for a blood sugar control agent and a feed pump for the
control agent and adapted to inject the control agent into the living body, and an arithmetic control unit including a microcomputer for calculating the blood sugar value from an output signal from the detecting unit and the dose of the control agent based on the value and controlling the pump of the injection unit in accordance with the result of calculation, the artificial pancreas being characterized in that an assembly including the blood sugar detecting unit and an assembly including the injection unit and the arithmetic control unit are separate from each other and are individually attachable to the body, the output signal of the detecting unit being adapted to be transmitted to the control unit by radio.

The term "to be attached to the body" as herein used includes two cases: direct attachment to the body, and attachment to a garment or the like.

According to the present invention, there is no need to collect the blood for the determination of blood sugar since the blood sugar detecting unit is provided with the measuring electrode means to be inserted into the living body. This lessens the burden on the patient, rendering the device continuously usable for a long period of time. The use of the measuring electrode means insertable into the body and the microcomputer makes the detecting unit and the arithmetic control unit small-sized, further making the device portable for use. Since the assembly including the detecting unit and the assembly including the injection unit and the control unit are separate from each other and are individually attached to the body, the device can be attached to the body with reduced limitations to the site of attachment and is easy to carry.

The present invention further provides a portable artificial pancreas which comprises a blood sugar detecting unit including measuring electrode means to be inserted into the living body for measuring the blood sugar of the living body, an injection unit including a container for a blood sugar control agent and a feed pump for the control agent and adapted to inject the control agent into the living body, and an arithmetic control unit including a computer for calculating the blood sugar value from an output signal from the detecting unit and the dose of the control agent based on the value and controlling the pump of the injection unit in accordance with the result of calculation, the artificial pancreas being characterized in that an assembly including the blood sugar detecting unit, an assembly including the injection unit and an assembly including the arithmetic control unit are separate from one another, the assembly including the blood sugar detecting unit and the assembly including the injection unit being individually attachable to the body, the output signal of the detecting unit being adapted to be transmitted to the control unit by radio, the control unit being adapted to transmit a control signal to the injection unit by radio. In addition to the foregoing adavantages, the device has the following advantages.

The assembly including the detecting unit, the assembly including the injection unit and the assembly including the control unit are separate from one another, and the first-mentioned assembly and the second-mentioned assembly can be attached to the body individually, while the third-mentioned assembly need not always be carried, so that the portable assemblies can be made further smaller and are portable with greater ease. When the assembly including the control unit is not carried about, it is possible to use a computer of large capacity for performing calculations with improved accuracy or to use the control unit for a plurality of portable artificial pancreas for centralized control.

Preferably the assembly including the blood sugar detecting unit comprises an arithmetic unit for calculating the blood sugar value from the output signal of the detecting unit and a blood sugar display for showing the result of calculation. It is then possible to attach this assembly only to the body during retiring for use as a blood sugar determination device, with the other assembly of relatively large size removed.

Other features of the present invention will become apparent from the following description.

Fig. 1 is an overall perspective view showing a first embodiment of the invention;

Fig. 2 is an overall block diagram;

Fig. 3 is a view in longitudinal section showing electrodes for measuring blood sugar values;

Fig. 4 is a graph showing the result of a glucose loading test conducted on canine blood with use of the electrode means of Fig. 3;

Fig. 5 is a side elevation showing a feed pump;

Fig. 6 is a plan view of the same;

Fig. 7 is an enlarged fragmentary view in section taken along the line VII-VII in Fig. 6;

Fig. 8 is an enlarged fragmentary view in section taken along the line VIII-VIII in Fig. 6;

Fig. 9 is an exploded perspective view showing the main components of the feed pump;

Fig. 10 is a block diagram showing the main construction of a pump drive circuit;

Fig. 11 is a block diagram showing a receiving assembly including an injection unit according to a second embodiment of the invention;

Fig. 12 is a block diagram showing a relay assembly including an arithmetic control unit;

Fig. 13 is a block diagram showing transmitters of a plurality of transmitting assemblies each including a blood sugar detecting unit, and a receiver of a relay assembly;

Fig. 14 is a time chart showing timer outputs of the receiver and the transmitters of Fig. 13;

Fig. 15 is a front view showing modified feed pumps;

Fig. 16 is a plan view partly broken away and showing the same; and

Fig. 17 is an enlarged view in section taken along the line XVII-XVII in Fig. 15.

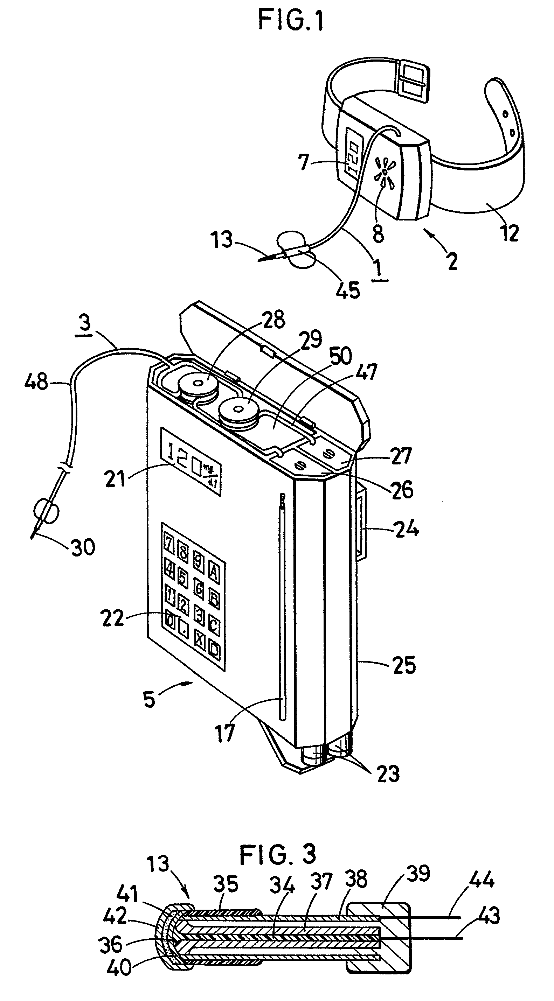

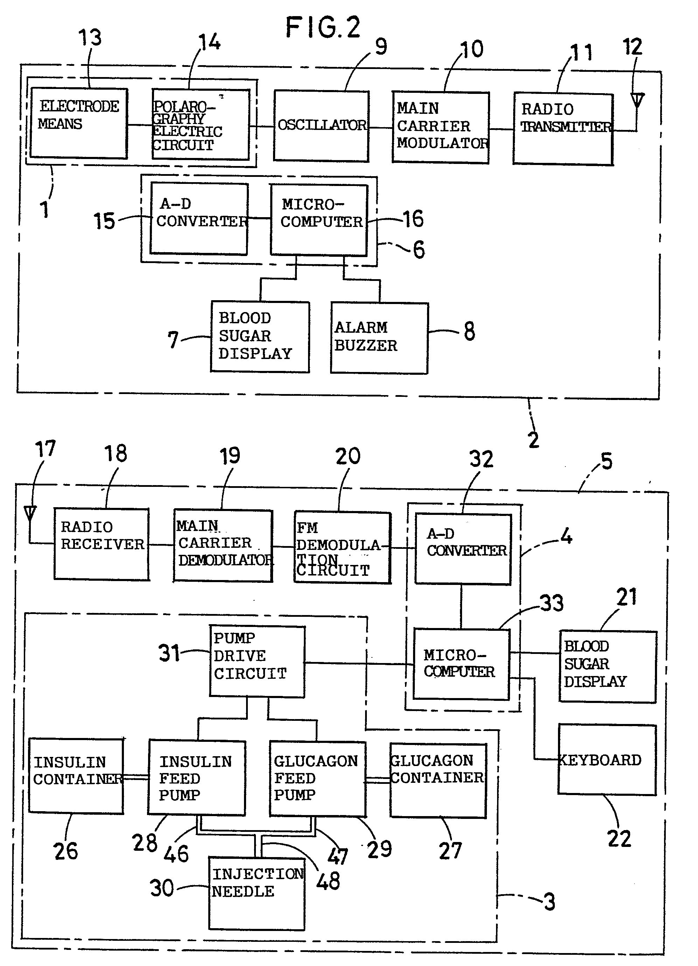

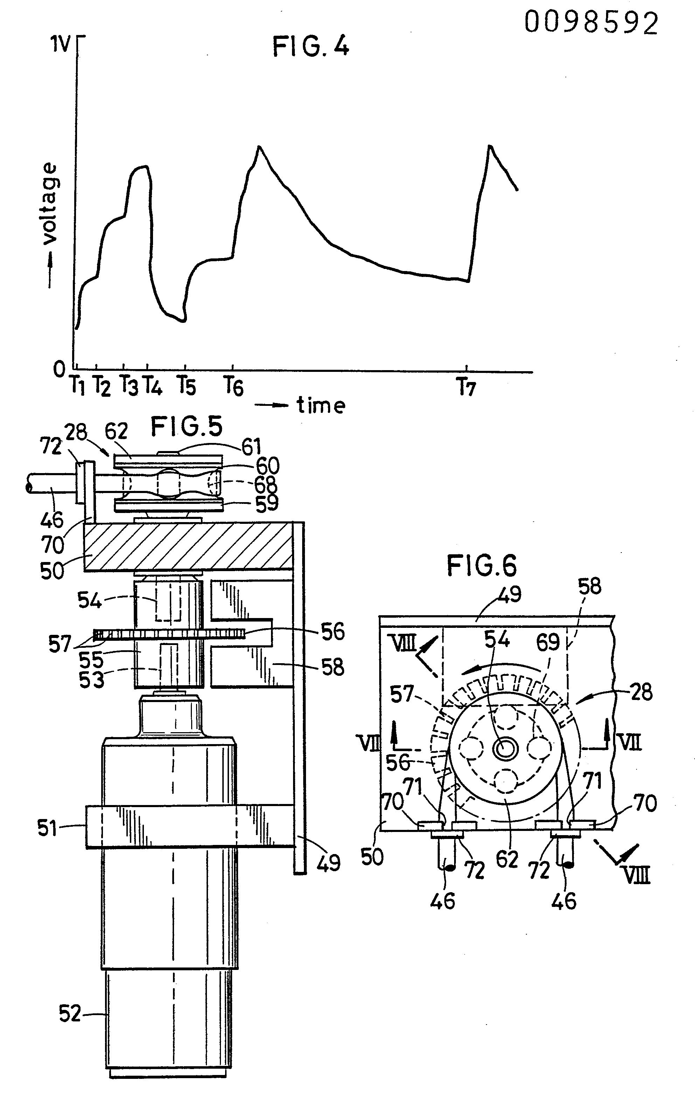

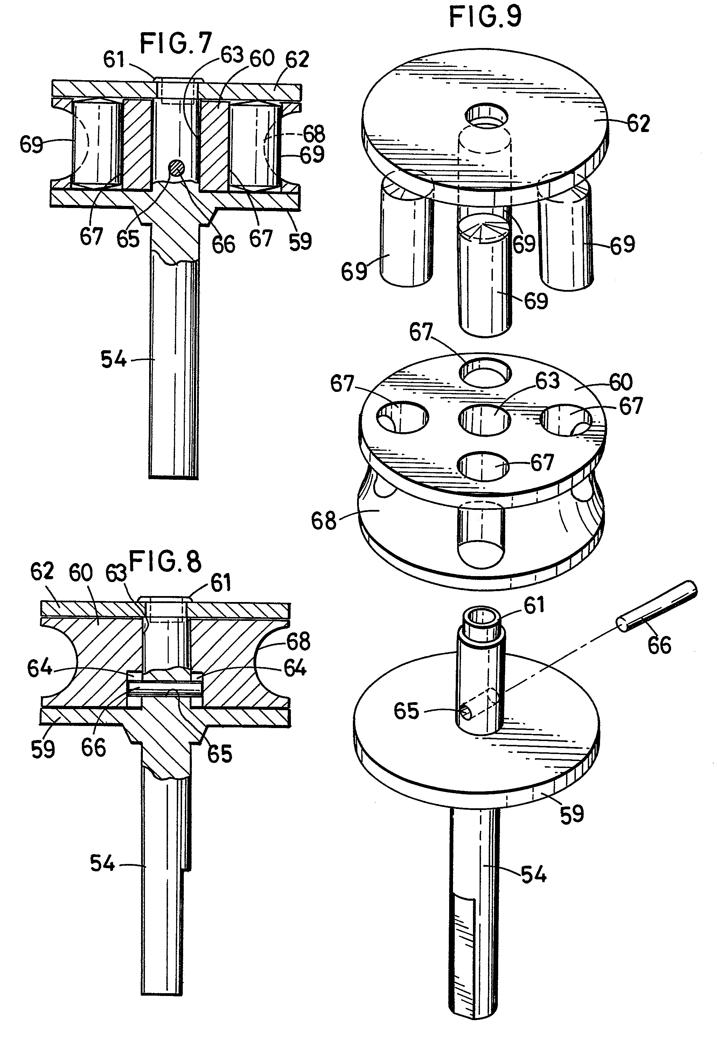

Figs. 1 to 10 show a first embodiment of the invention. As seen in Figs. 2 and 3, the portable artificial pancreas comprises a watch-shaped transmitting assembly 2 including a blood sugar detecting unit 1, and a receiving assembly 5 including an injection unit 3 and an arithmetic unit 4.

The transmitting assembly 2 includes, in addition to the detecting unit 1, an arithmetic unit 6, a blood sugar dispaly 7 of the liquid crystal type, an alarm buzzer 8, a voltage-controlled oscillator 9, a main carrier modulator 10, a radio transmitter 11 and a transmitting aerial 12. The band portion of the assembly 2 serves as the transmitting aerial. The blood sugar detecting unit 1 comprises electrode means 13 for measuring blood sugar values by polarography and a polarography electric circuit 14. The arithmetic unit 6 comprises an A-D converter 15 and a microcomputer 16.

The receiving assembly 5 includes, in addition to the injection unit 3 and the control unit 4, a receiving aerial 17, a radio receiver 18, a main carrier demodulator 19, an FM demodulation circuit 20, a blood sugar display 21 of the liquid crystal type and a keyboard 22. These components and power supply dry batteries 23 are housed in a single small casing 25 provided with a band retainer 24. The injection unit 3 comprises an insulin container 26, a glucagon container 27, an insulin feed pump 28, a glucagon feed pump 29, an injection needle 30 and a pump drive circuit 31. The arithmetic control unit 4 comprises an A-D converter 32 and a microcomputer 33.

The electrode means 13 is adapted to measure blood glucose concentrations based on current resulting from the electrolysis of hydrogen peroxide which is produced from glucose in the presence of glucose oxidase serving as a catalyst. As seen in Fig. 3 in greater detail, the electrode means 13 comprises a platinum electrode (anode) 34 and a silver electrode (cathode) 35. The platinum electrode 34 is insulated with glass 37 except at its front end 36 serving as an electrode reaction portion and at its rear end serving as a lead wire connecting portion. The platinum electrode 34 is inserted in a stainless steel cylinder 38 having the silver electrode 35 formed over its outer surface. The front ends of the platinum electrode 34 and the cylinder 38 are fixed together by thermally adhering the front end of the glass insulator 37 to the front end of the cylinder 38. The rear ends of the platinum electrode 34 and the cylinder 38 are fixed together by a plastic plug 39 fitting over these rear ends. A cellulose acetate film 40 serving as a hydrophilic semipermeable membrane which is permeable to hydrogen peroxide is laminated to the surface of the front end 36 of the platinum electrode 34, with an immobilized glucose oxidase membrane 41 further laminated to the outer surface of the film 40. A polyurethane film 42 serving as a hydrophilic semipermeable membrane which is permeable to glucose, oxygen and hydrogen peroxide is laminated to the membrane 41. The platinum electrode 34 and the cylinder 38 are connected, at their rear ends, to insulated lead wires 43 and 44, respectively. The measuring electrode means 13 is attached to the forward end of a catheter 45 to be lodged in vivo and is inserted into the blood vessel or beneath the skin. The signal from the electrode means 13 is fed to the polarography electric circuit 14 through the catheter 45. The circuit 14 applies a predetermined voltage across the electrodes 34 and 35 to measure the current resulting from the electrolysis of hydrogen peroxide and amplifies the current. The blood glucose concentration, i.e., blood sugar value, is determined based on the amplified output signal.

Fig. 4 shows the result of a glucose loading test conducted on canine blood with use of the electrode means 13. The measurements well coincides with those obtained by the standard determination method generally practiced at present. With reference to Fig. 4, the time is plotted as abscissa vs. the voltage as ordinate. The electrode means is exposed to standard test solutions of 100 mg/dℓ from T1 to T2, of 200 mg/dℓ from T2 to T3 and of 300 mg/dℓ from T3 to T4, to physiological saline from T4 to T5, and to normal canine blood from T5 to T6. From T6 to T7, 5 cc of 50% aqueous glucose solution is intravenously given to the thigh of the dog.

Further the results of basic experiments have revealed that the electrode means 13 operates stably in vitro systems for more than 24 hours, exhibiting linear characteristics in response reaction at glucose concentrations of 0 to 500 mg/dℓ, a short response time of the order of seconds and good stability at temperatures of 36 to 42° C. _In_ _vivo_ animal experiments have demonstrated that the electrode means exhibits good responsiveness to the load of glucose and insulin hypoglycemia. Thus it has been ascertained that the electrode means affords highly reliable measurements.

The components of the measuring electrode means 13 can also be made of suitable materials other than those mentioned above, while it is preferable to use platinum, gold or like noble metal for the anode and to use silver, silver chloride or the like for the cathode. Preferred materials for the insulator are glass, plastics, etc. Cellulose derivatives such as cellulose mono-, di- or triacetate, cellulose propionate and cellulose butyrate are desirable for forming the hydrophilic semipermeable membrane which is permeable to hydrogen peroxide. The carrier for immobilizing glucose oxidase is preferably porous glass or plastics. It is desirable to incorporate heparin or like anticoagulant into the hydrophilic semipermeable membrane permeable to glucose, oxygen and hydrogen peroxide for the prevention of blood coagulation on the surface of the electrode means. Glucose oxidase may be immobilized and laminated by repeating the procedure of immersing the front end of the electrode means in a glucose oxidase-containing liquid and drying. To render the electrode means flexible, a plastic tube may be used instead of the stainless steel cylinder 38. In this case, the cathode can be formed by forming a silver film on the outer surface of the plastic tube by vacuum evaporation and further plating the film with silver.

The microcomputer 16 of the arithmetic unit 6 has a programmable read-only memory (PROM) having stored there in a program for calculating blood sugar values based on output signals from the blood sugar detecting unit 1.

The insulin container 26 is filled with insulin, and the glucagon container 27 with glucagon. Liquid feeding elastic tubes 46 and 47 are connected, each at its one end, to the containers 26 and 27. The two tubes 46 and 47 are joined into a single tube 48, which extends outward from the casing 25 and is connected to the single injection needle 30.

Figs. 5 to 9 show the insulin feed pump 28 in greater detail. A pump support plate 49 fixed vertically to the casing 25 is fixedly provided one one side thereof with a horizontal bracket 50 and another horizontal bracket 51 positioned below and opposed to the bracket 50. A d.c. motor 52 equipped with a reduction gear and oriented upward is fixed to the lower bracket 51. A vertical rotary shaft 54 coaxial with the motor shaft 53 is rotatably supported at an intermediate portion by the upper bracket 50. The motor shaft 53 and the rotary shaft 54 are connected to each other by a coupling 55. A rotary disk 56 fixed horizontally to the coupling 55 has 24 cutouts 57 formed in its circular outer peripheral portion and arranged equidistantly. Mounted on an upper portion of the support plate 49 on one side thereof is a photoelectric rotational angle sensor 58 comprising a light-emitting element and a photocell arranged above and below the peripheral portion of the disk 56. A roller retainer 59 in the form of a horizontal disk and positioned immediately above the upper bracket 50 is integral with the rotary shaft 54. A roller holder 60 in the form of a short solid cylinder is fitted to the portion of the rotary shaft 54 above the retainer 59. The upper end of the rotary shaft 54 projecting upward beyond the roller holder 60 is in the form of a hollow cylinder 61 having a thin wall and slightly smaller diameter than the other shaft portion. A disk-like roller retainer 62 is fitted around the cylindrical portion 61 and secured to the rotary shaft 54 by enlarging the upper end of the cylindrical portion 61 outward. The roller holder 60 is made of polytetrafluoroethylene and formed in the center of its bottom with a groove 64 extending diametrically slightly beyond opposite sides of the rotary shaft inserting bore 63. A horizontal bore 65 extending through the rotary shaft 54 is formed in the portion thereof corresponding to the groove 64. A horizontal pin 66 is inserted through the bore 65. Opposite ends of the pin 66 projecting from the rotary shaft 54 fit in the groove 64. The rotation of the rotary shaft 54 is delivered to the roller holder 60 by the pin 66. Four circular bores 67 extending through the roller holder 60 vertically are equidistantly arranged on a circumference centered about the rotary shaft 54. The roller holder 60 has an annular tube guide groove 68 having a semicircular cross section, formed in its outer peripheral side surface over the entire circumference thereof and partly positioned in the bores 67. A solid cylindrical roller 69 having an outside diameter slightly smaller than the inside diameter of the circular bore 67 is rotatably fitted in each bore 67. The outer periphery of the roller 69 is partly positioned in the guide groove 68. Each of the upper and lower ends of the roller 69 is in the form of a cone having a very small height. The length of the roller 69 is slightly smaller than the distance between the upper and lower roller retainers 62 and 59. The roller 69 is substantially immovable axially thereof. The rotary shaft 54, the roller retainer 62 and the rollers 69 are made of stainless steel. The intermediate portion of the liquid feeding elastic tube 46 connected to the insulin container 28 is reeved around the portions, positioned within the guide groove 68, of the rollers 69 disposed in the range of about 180$^{O}$ about the center of the roller holder 60 and is tensioned, in the following manner. Two tube holding plates 70 are fixed to the upper side of one end of the upper bracket 50, with a U-shaped cutout 71 formed in the upper portion of each plate 70. On the other hand, the intermediate portion of the tube 46 has two annular stoppers 72 spaced apart, secured to the tube by suitable means as by adhesion and having a larger outside diameter than the tube 46. The tube portion between the stoppers 72 is reeved around the rollers 69, the tube 46 is pulled, and the portions of tube adjacent the stoppers 72 and positioned closer to the rollers 69 are inserted into the cutouts 71 of the holding plates 70, with the stoppers 72 in pressing contact with the plates 70, whereby the tube 46 is reeved around the rollers 69 under predetermined tension. By the rotation of the roller holder 60 in the direction of arrow shown in Fig. 6, insulin is supplied from the container 26 to the needle 30 through the tube 46 as indicated by arrows in Fig. 6. The amount of supply is in proportion to the angle of rotation of the pump 28, i.e., the roller holder 60.

The glucagon feed pump 9 also has the same construction as above.

The microcomputer 33 of the arithmetic control unit 4 has a PROM having stored therein a program for calculating blood sugar values from output signals sent forward from the detecting unit 1 by radio as will be described later and also calculating doses of insulin and glucagon from the blood sugar values and for controlling the pumps 28 and 29 of the injection unit 3 based on the result of calculation. The microcomputer 33 has a random access memory (RAM) for storing calculation parameters and measuring data. The calculation parameters vary with the body weight of the patient, the concentration of insulin and the like. Such parameters, measuring intervals, etc. are set with use of the keyboard 22.

To use the portable artificial pancreas, the transmitting assembly 2 is fastened to the arm of the patient, with the measuring electrode means placed into a blood vessel or beneath the skin, and the receiving assembly 5 is fastened to a band or the like on the patient, with the injection needle 30 placed into a blood vessel or beneath the skin. Blood sugar is measured at a specified time interval (e.g. every minute), and the doses, i.e., the amounts of insulin and glucagon to be injected, are calculated based on the measurements. The required amount of insulin or glucagon is injected.

More specifically stated, the analog signal from the electrode means 13 is amplified by the polarography electric circuit 14 and then converted by the A-D converter 15 of the arithmetic unit 6 to a digital signal, which is fed to the micrcomputer 16. At a given time interval, the microcomputer 16 calculates the blood sugar value based on the digital signal and displays the value on the display 7. When the blood sugar value exceeds a predetermined upper limit or lower limit, the buzzer 8 gives an alarm. On the other hand, the polarography circuit 14 gives an output signal to the voltage-controlled oscillator 9 for voltage-frequency conversion, and the resulting output is fed to the main carrier modulator 10 and then to the transmitter 11, from which the output is transmitted through the aerial 12.

The signal sent out from the transmitting assembly 2 is received by the receiver 18 of the receiving assembly 5 via the aerial 17 and fed through the main carrier demodulator 19 to the FM demodulation circuit 20, by which the signal is converted to an analog signal in proportion to the output signal of the polarography electric circuit 14. The analog signal is converted by the A-D converter 32 of the arithmetic control unit 4 to a digital signal, which is sent to the microcomputer 33. At a specified time interval, the microcomputer 33 calculates the blood sugar value based on the digital signal, shows the value on the display 21 and calculates the doses of insulin and glucagon based on the result of calculation. The method of calculation is the same as is practiced in known bedside artificial

18                    0098592

pancreases and will not be described.  At least one of
the doses of insulin and glucagon thus obtained is zero;
only one of insulin and glucagon or neither of them is
administered in accordance with the blood sugar level.
When there is a need to inject insulin or glucagon, the
microcomputer 33 calculates the angle of rotation of the
pump 28 or 29 corresponding to the amount to be injected,
i.e., dose, giving instructions to the pump drive circuit
31 as to the pump to be driven and the rotational angle.

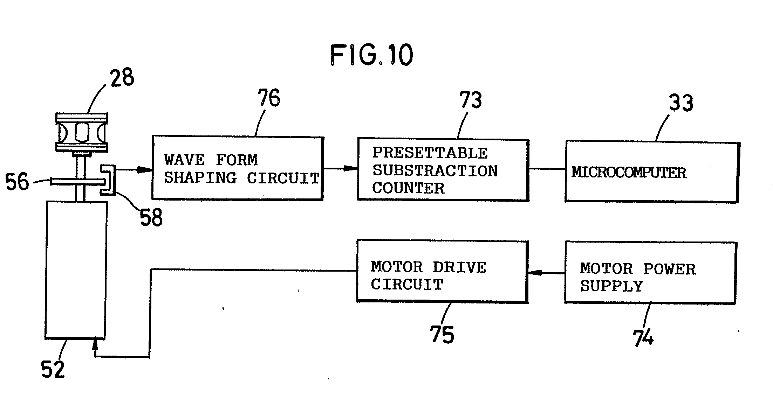

Fig. 10 shows the main construction of the pump drive
circuit 31.  For example when insulin is to be injected, the
pump 28 is rotated only through the angle instructed by the
microcomputer 33 in the following manner.  The rotational
angle instruction is given by the microcomputer 33 in terms
of the number of pitches of the cutouts 57 in the rotary disk
56,   one pitch ($15^{\circ}$) being one unit, and the instruction
value is set on a presettable subtraction counter 73.  For
example when the pump 28 needs to be rotated one turn, an
instruction value of 24 is set on the counter 73, whereupon
a motor drive circuit 75 extending from a motor power supply
74 to the motor 52 is closed to start the pump 28, permitting
the disk 56 to rotate with the pump 28.  Every time the
pump 28, i.e., the disk 56, rotates by one pitch of cutout
57, the rotational angle sensor 58 feeds a signal to the
counter 73 via a wave form shaping circuit 76.  Every time such a signal is given, 1 is subtracted from the contents of the counter 73. While the contents of the counter 73 remain positive, the pump 28 continues rotation. Upon the reduction of the contents of the counter 73 to zero, the motor drive circuit 75 opens to stop the pump 28. In this way, the pump 28 rotates by an amount corresponding to the instruction value given by the microcomputer 33, whereby a specified quantity of insulin in proportion to that amount is infused or given subcutaneously from the container 26 via the tubes 46, 48 and needle 30. Glucagon is administered in the same manner as above.

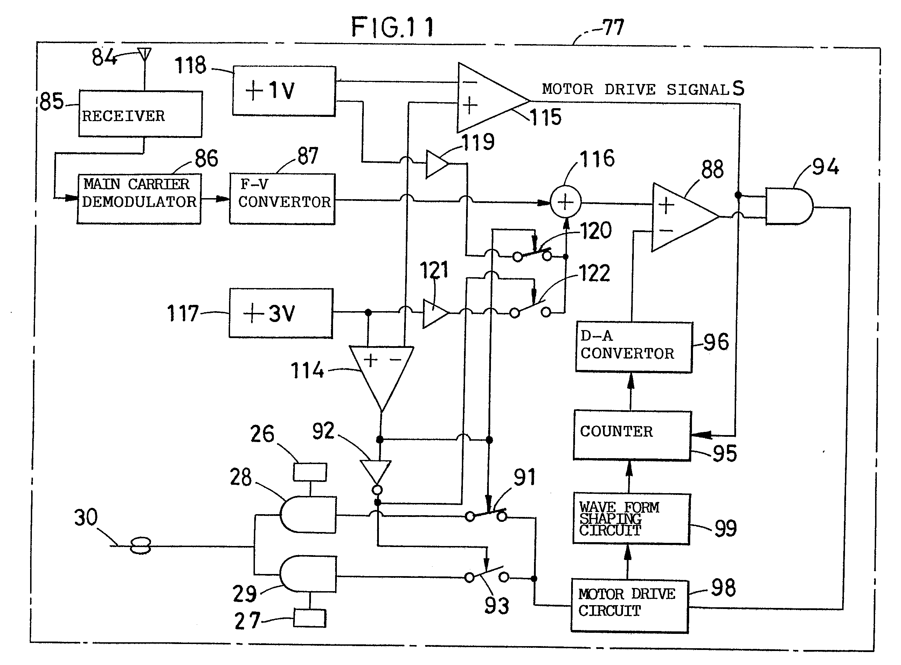

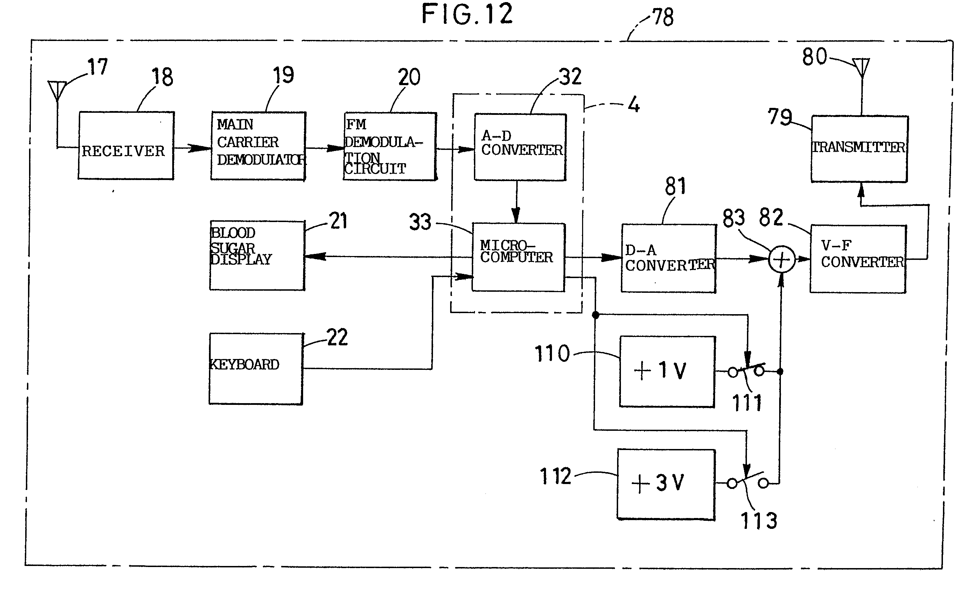

Figs. 11 and 12 show a second embodiment of the invention. The portable artificial pancreas comprises a transmitting assembly including a blood sugar detecting unit, a receiving assembly 77 including an injection unit, and a relay assembly 78 including an arithmetic control unit 4.

The transmitting assembly is the same as the transmitting assembly 2 of the first embodiment. Fig. 11 shows the construction of the receiving assembly 77. Throughout the first and second embodiments, like parts are referred to by like reference numerals. The receiving assembly 77 is housed in the same casing as used for the first embodiment and is fastened, for example, to the belt worn by the patient. Fig. 12 shows the construction of the relay assembly 78. The relay assembly 78 is usually housed in a casing and can be carried about by the patient independently of the transmitting assembly 2 and the receiving assembly 77, or can be installed in a nurse station or the like. In the latter case, a computer of larger capacity than is the case with the first embodiment can be used for the arithmetic control unit for performing calculations with improved accuracy, or when a time division system is employed, the arithmetic control unit can be used commonly for a plurality of portable artificial pancreases under centralized or cencentrated control.

The second embodiment operates in the same manner as the first in respect of the transmitting assembly 2, and the receiver 18 through the arithmetic control unit 4 of the relay assembly 78. When the pump to be driven and the angle of rotation thereof are determined by the microcomputer 33 of the control unit 4, the information is sent to a transmitter 79 in timed relation with a timer for determining the measuring interval. The data is sent out from an aerial 80 in the form of an FM signal. The rotational angle data or instruction is the same as is the case with the first embodiment. The rotational angle signal given by the microcomputer 33 is converted by a D-A converter 81 to a 0.005 V analog signal per unit of instruction value (angle of rotation, $15^{\circ}$, of the pump 28 or 29). Since the D-A converter is adapted for eight bits, the maximum instruction value for the rotational angle is 256 ($=2^8$). Since the output thereof is 0.005 V per unit instruction value, the output voltage of the converter 81 is 0.005 V for the minimum instruction value (=1) or 1.28 V for the maximum (=256). Further when the insulin pump 28 is to be driven in response to an instruction signal from the microcomputer 33, an analog switch 111 of a 1 V reference voltage generator 110 is closed to deliver 1 V, or when the glucagon pump 29 is to be driven, an analog switch 113 of a 3 V reference voltage generator 112 is closed to give 3 V, each as a pump selecting signal. The selecting signal is added to the output of the D-A converter 81 in an operational amplifier 83. Accordingly the output voltage of the operational amplifier 83 is 1.005 (=1+0.005) to 2.28 (=1+1.28) V when rotating the insulin pump 28 by 1 to 256 units, or is 3.005 (=3+0.005) to 4.28 (=3+1.28) V when rotating the glucagon pump 29 by 1 to 256 units. The output of the amplifier 83 is subjected to voltage-frequency conversion into a signal of 1000 Hz per volt by a V-F (voltage-frequency) converter 82. Thus the frequency of the converted signal is 1005 to 2280 Hz for driving the insulin pump 28, or 3005 to 4280 Hz for driving the glucagon pump 29. The output signal from the V-F converter 82 is finally subjected to FM modulation by the transmitter 79 and sent out from the aerial 80. With the system described, the pump rotational angle signal and pump selecting signal are combine together, and the composite signal is modulated into a subcarrier (1005 to 4280 Hz) and further into a radio wave of the HF band. The FM-FM system therefore assures transmission of accurate information to the receiving assembly 77.

The receiving assembly 77 receives the signal thus forwarded from the relay assembly 78 and controls the pumps 28 and 29 of the injection unit in response to the signal in the following manner. The signal received by a receiver 85 by way of an aerial 84 is fed through a main carrier demodulator 86 to a F-V (frequency-voltage) converter 87 and thereby converted to a voltage, which is fed to a pump selecting comparison circuit 114, a pump driving comparison circuit 115 and an operational amplifier 116. The pump selecting comparison circuit 114 is given a reference voltage of 3 V by a 3 V reference voltage generator 117, while a reference voltage of 1 V is applied to the pump driving comparison circuit 115 by a 1 V reference voltage generator 118. The generator 118 is connected to an operational amplifier 116 via a polarity reversing operational amplifier 119 and an analog switch 120. The generator 117 is connected to the amplifier 116 via a polarity reversing amplifier 121 and an analog switch 122. The output voltage of the F-V converter 87 corresponds to that of the operational amplifier 83 of the relay assembly 78 and is 1.005 to 2.28 V when driving the insulin pump 28 or 3.005 to 4.28 V when driving the glucagon pump 29. Accordingly when the insulin pump 28 is to be driven, the output of the pump selecting comparison circuit 114 becomes high (hereinafter mentioned as "H"), and an analog switch 91 provided for the insulin pump 28 and connected directly to the circuit 114 and the analog switch 120 for the voltage generator 118 only are closed. Consequently -1 V is added in the operational amplifier 116 to the output (1.005 to 2.28 V) of the F-V converter 87, so that the amplifier 116 gives an output voltage (0.005 to 1.28 V) corresponding to the output of the D-A converter 81 of the relay assembly 78 and in proportion to the angle through which the pump 28 is to be rotated. Conversely when the glucagon pump 29 is to be driven, the output of the pump selecting comparison circuit 114 becomes low (hereinafter mentioned as "L"), with the result that only an analog switch 93 connected to the circuit 114 via an inverter 92 and provided for the glucagon container 29 and the analog switch 122 are closed. Therefore, -3 V is added to the output (3.005 to 4.28 V) of the F-V converter 87 in the amplifier 116, whereupon the amplifier 116 also gives an output voltage (0.005 to 1.28 V) corresponding to the output of the D-A converter 81 of the relay assembly 78 and in proportion to the angle through which the pump 29 is to be rotated. The output of the operational amplifier 116 serves as a reference voltage for a rotational angle controlling comparison circuit 88. On the other hand, when a signal is applied to the main carrier demodulator 86, the output of the F-V converter 87 is not lower than 1.005 V, so that the motor drive signal S delivered from the output terminal of the pump driving comparison circuit 115 to an input terminal of an AND circuit 94 and to the resetting terminal of an 8-bit binary counter 95 turns H to reset the counter 95. Upon resetting, the counter 95 feeds an analog voltage of 0 V to the comparison circuit 88 via a D-A converter 96. With a signal at H level given by the output terminal of the circuit 88 to the AND circuit 94, the output of the AND circuit 94 turns H, whereupon a motor drive circuit 98 is energized, driving the insulin pump 28 in the case of Fig. 11. Every time the pump 28 rotates by one pitch of cutout 57, the rotational angle sensor 58 feeds a pulse signal to the counter 95 via a wave form shaping circuit 99. Every time such a signal is given, 1 is added to the contents of the counter 95, giving an increment of 0.005 V to the output of the D-A converter 96. While the output of the converter 96 is smaller than the reference voltage, the pump 28 continues rotation, but upon the output matching the reference voltage, the output of the comparison circuit 88 turns L, turning the output of the AND circuit 94 also L and consequently de-energizing the motor drive circuit 98 to stop the pump 28. The reference voltage of the comparison circuit 88, i.e., the output of the operational amplifier 116, corresponds to the rotational angle instruction value for the pump 28, while the output of the D-A converter 96 corresponds to the actual angle of rotation of the pump 28. Thus the pump 28 rotates by an amount corresponding to the value instructed by the control unit 4. In this case, only while the output of the F-V converter 87 is at least 1 V with an input applied to the main carrier demodulator 86, the motor drive signal S, i.e., the input to the AND circuit 94 is H. This eliminates errors in the operation of the pumps 28 and 29. Glucagon is injected in the same manner as above.

When a single arithmetic control unit is installed, for example, in a nurse station for use with a plurality of portable artificial pancreas, in other words, when a single relay assembly 78 is provided for sets of transmitting assemblies 2 and receiving assemblies 77, there is the need to process the signals from the transmitters 11 of the plurality of transmitting assemblies 2 by the single receiver 18 of the relay assembly 78. Generally a frequency division system or a time division system will be used for treating the signals from a plurality of transmitters by a single receiver. In the case of the frequency division system, the transmitters are assigned different carrier frequencies, while the receiver is provided with demodulation circuits for the carrier frequencies. Although this system can be adapted to simultaneously measure the blood sugar values of a plurality of living bodies in real time, the demodulation circuits needed are equal in number to the number of the transmitters, rendering the circuit construction of the receiver complex. In the case of the time division system, the transmitters each have a timer for determining the transmitting time to repeatedly transmit radio waves in a predermined order. When handling signals of high frequency components (0 to several kilohertz) such as electrocardiogram, electromyogram and electroencephalogram, this system is unable to perform real-time continuous measurements, and the frequency division system must therefore be resorted to, but blood sugar values can be handled by the time division system without any substantial problem since the variations in signals are very gentle. The time division system, in which the same frequency is usable for different transmitters, needs only to have a single demodulation circuit, which makes the receiver simple in circuit construction. However, the system has the following drawback. With the time division system, the timers incorporated in the transmitters operate each independently, so that when the system is used for a long period of time, signals from different transmitters are likely to overlap to cause interference even if precision timers such as quartz oscillators are used because the degree of precision is limited.

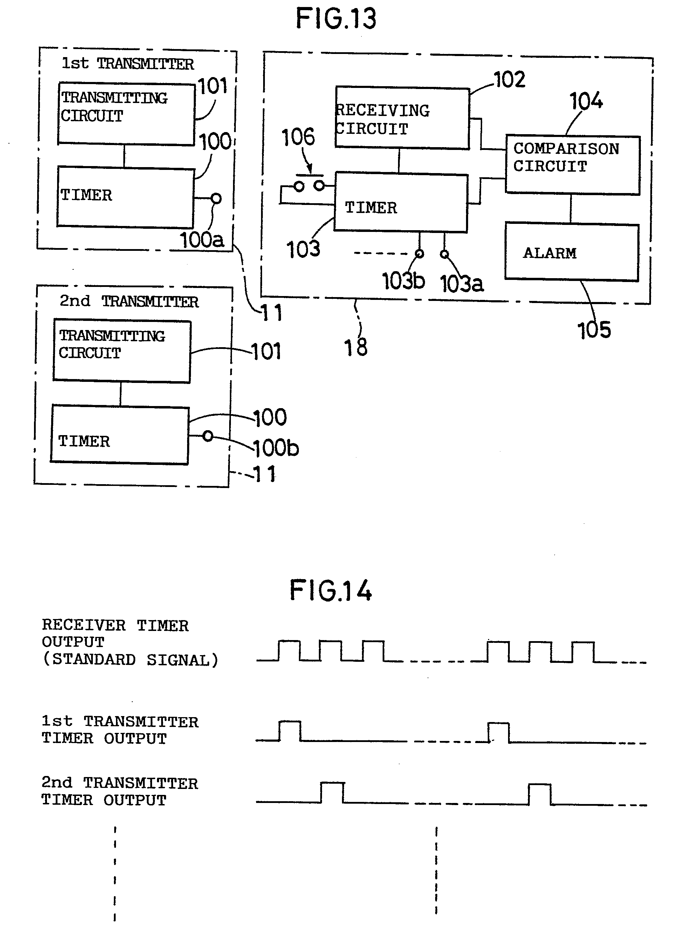

Accordingly, in the case where a single arithmetic control unit is provided for a plurality of portable artificial pancreas with use of the time division system, such that the transmitters 11 of a plurality of transmitting assemblies 2 transmit data to the receiver 18 of a single relay assembly 78, it is desirable to construct the transmitters 11 and the receiver 18 as shown in Fig. 13 to avoid the above-mentioned interference.

With reference to Fig. 13, each transmitter 11 of the transmitting assembly 2 comprises a timer 100 and a transmitting circuit 101. The receiver 18 of the relay assembly 78 comprises a receiving circuit 102, timer 103, comparison circuit 104 and alarm 105.

The plurality of transmitters 11 and the receiver 18 operate in the following manner.

Before operation, the resetting terminal 100a of the timer 100 of the first transmitter 11 is connected to a first resetting terminal 103a of the timer 103 of the receiver, the resetting terminal 100b of the timer 100 of the second transmitter 11 is connected to a second resetting terminal 103b of the timer 103 of the receiver 18, and the resetting terminals of the timers of the other transmitters are similarly connected to the corresponding resetting terminals of the timer 103 of the receiver 18. After depressing a start switch 106 for the timer 103 in this state, the timers 100 of the transmitters 11 are disconnected from the timer 103 of the receiver 18. The depression of the start switch 106 brings the timers 100 and 103 into timed relation, such that as seen in Fig. 14, the timers 100 of the respective transmitters 11 deliver an output for a specified period of time at a predetermined time interval, permitting each transmitter 11 to deliver a signal during the time period (transmitting time). There is a given time lag between the transmitting periods of the transmitters 11, so that the plurality of transmitters 11 send out data in a predetermined order in repetition. On the other hand, the timer 103 of the receiver 18 emits reference or standard signals in corresponding relation to the transmitting periods of the respective transmitters 11 as shown in Fig. 14, such that every time a signal is received from each transmitter 11, the comparison circuit 104 checks the received signal and the corresponding standard signal for the possible time lag. Immediately after the start switch 106 is depressed for resetting as stated above, there will be no time lag between the two signals, but if there is an error between the timers 100 and 103 relative to each other, a time lag occurs between the received signal and the standard signal with the lapse of time. Before the lag reaches a predetermined value, the receiver continues to receive signals since there is no likelihood of interference. The lag, when exceeding the predetermined value, indicates a likelihood of interference, so that the comparison circuit 104 feeds a signal to the alarm 105 to give warning with a buzzer or lamp. If such an alarm is given, the aforementioned resetting procedure is followed for the timers 100 and 103, whereby the time lag between the received signal and the standard signal is eliminated to avoid interference.

The measuring electrode means to be inserted into the living body is not limited to the one included in the above embodiment but can be modified suitably.

The feed pump of the foregoing embodiment can be made smaller than the conventional one, composed of a smaller number of parts and fabricated less expensively because the rollers are fitted in the circular bores of the roller holder without the necessity of being supported by roller bearings. Further because the elastic tube is reeved around the portions of the rollers positioned in a guide groove which is formed in the peripheral side surface of the holder and partly positioned in, or sharing the same space as, the circular bores, the tube can be effectively prevented from zigzagging and thereby given a prolonged life without the need to use rollers of special shape or tube guides conventionally employed. The d.c. motor used for the feed pump of the foregoing embodiment has the advantage of being lesser in power consumption than pulse motors. However, the feed pump and the pump drive circuit are not limited in construction to those of the embodiment but can be modified suitably.

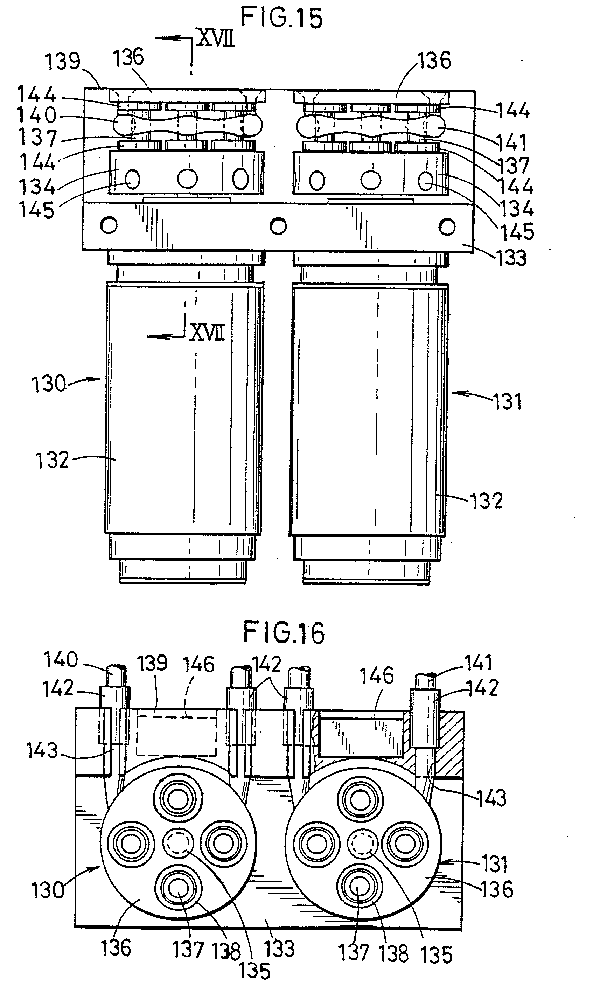

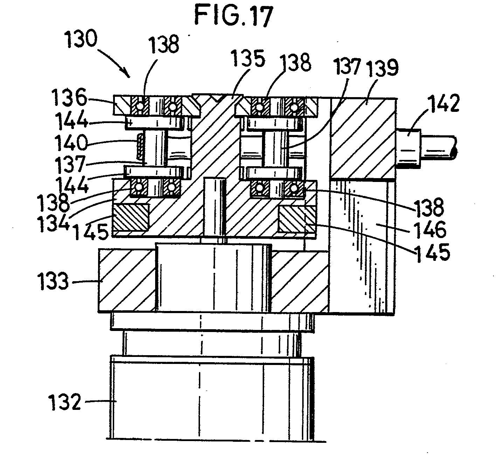

Figs. 15 to 17 show an insulin feed pump 130 and a glucagon feed pump 131 which differ from those of the foregoing embodiments. Each of these pumps 130 and 131 has a d.c. motor 132 equipped with a reduction gear and fixed to a horizontal bracket 133 as oriented upward. Coupled to the shaft of the motor 132 is a vertical rotary shaft 135 integral with a roller support 134 in the form of a horizontal circular flange. A disk-like roller support 136 fixed to the upper end of the rotary shaft 135 is opposed to the roller support 134 as spaced apart therefrom by a distance. Four rollers 137 are arranged in parallel with the rotary shaft 135 and equidistantly spaced apart on a circumference centered about the rotary shaft 135. Each roller 137 is rotatably supported at its upper and lower ends by roller bearings 138 mounted on the upper and lower roller supports 136 and 134. An elastic tube holder 139 is fixed to one end of the bracket 133. Intermediate portions of two liquid feeding

0098592

elastic tubes 140, 141 are reeved, under tension, around some of the rollers 137 of the pumps 130, 131 with use of stoppers 142 secured to the tubes and four stepped grooves 143 formed in the upper side of the holder 139. A flange 144 is formed at the upper and lower ends of each roller 137 integrally therewith for preventing the tube 140 or 141 from zigzagging and slipping off. Eight permanent magnets 145 equidistantly spaced apart are embedded in the outer pripheral side of the roller support 134 of each rotary shaft 135. On the other hand, the portions of the tube holder 139 to be opposed to the permanent magnets 145 are cut out, and rotational angle detectors 146 each comprising a magnetic sensor are fixed to the cutout portions. In this case, every time the rotary shaft 135 of each of the pumps 130, 131 rotates by an amount corresponding to one pitch ($45^{\circ}$) of magnet 145, the magnetic sensor of the detector 146 emits a signal. The microcomputer 33 gives a rotational angle instruction in terms of the number of units, one unit being $45^{\circ}$. The rotation of the pumps 130, 131 is controlled based on such instruction values and output signals from the detectors 146. With the exception of the above feature, the modification is the same as the foregoing embodiments.

The front ends of the two liquid feeding elastic tubes, although connected to a single injection needle according to the above embodiments, may be connected to two injection needles individually.

    While the injection units of the above embodiments comprise an insulin container, glucagon container, insulin feed pump and glucagon feed pump to administer insulin or glucagon to the living body, the injection unit may be adapted to inject only one of insulin and glucagon into the body.  Furthermore, glucose may be given in place of glucagon.

0098592

CLAIMS

1. A portable artificial pancreas comprising a blood sugar detecting unit including electrode means to be inserted into the living body for measuring the blood sugar of the living body, an injection unit including a container for a blood sugar control agent and a feed pump for the control agent and adapted to inject the control agent into the living body, and an arithmetic control unit including a microcomputer for calculating the blood sugar value from an output signal from the detecting unit and the dose of the control agent based on the value and controlling the pump of the injection unit in accordance with the result of calculation, the artificial pancreas being characterized in that an assembly including the blood sugar detecting unit and an assembly including the injection unit and the arithmetic control unit are separate from each other and are individually attachable to the body, the output signal of the detecting unit being adapted to be transmitted to the control unit by radio.

2. A portable artificial pancreas as defined in claim 1 wherein the assembly including the blood sugar detecting unit further comprises an arithmetic unit for calculating the blood sugar value from the output signal of the detecting unit and a blood sugar display for showing the result of calculation.

3. A portable artificial pancreas as defined in claim 1 wherein the injection unit comprises a container for a hypoglycemic agent, a container for a hyperglycemic agent, a feed pump for the hypoglycemic agent and a feed pump for the hyperglycemic agent and is adapted to inject the hypoglycemic agent or the hyperglycemic agent into the living body, and the arithmetic control unit calculates the doses of the hypoglycemic agent and the hyperglycemic agent based on the result of calculation of the blood sugar value and controls the pumps of the injection unit in accordance with the result of calculation of the doses.

4. A portable artificial pancreas as defined in any one of claims 1 to 3 wherein the electrode means comprises a bar-like anode insulated except at its front end serving as an electrode reaction portion and at its rear end serving as a lead wire connecting portion, and a cylindrical cathode having the anode fixedly provided therein, and the anode is cover over the surface of its front end with a hydrophilic semipermeable membrane permeable to hydrogen peroxide, then with an immobilized glucose oxidase membrane and further with a hydrophilic semipermeable membrane permeable to glucose, oxygen and hydrogen peroxide by lamination.

5. A portable artificial pancreas as defined in claim 4 wherein the cathode is formed on the outer surface of a cylinder, and the anode is inserted in the cylinder and provided with an insulator fixed at its front end to the front end of the cylinder, the anode and the cathode cylinder being fixed at their rear ends to a plastic plug.

6. A portable artificial pancreas as defined in claim 5 wherein the anode is a noble metal electrode insulated with glass or plastic except at its front end and rear end, and the cathode is a silver electrode or silver chloride electrode formed on the outer surface of a metal cylinder or plastic tube.

7. A portable artificial pancreas as defined in claim 4 wherein the hydrophilic semipermeable membrane permeable to hydrogen peroxide is a cellulose acetate film.

8. A portable artificial pancreas as defined in claim 4 wherein the hydrophilic semipermeable membrane permeable to glucose, oxygen and hydrogen peroxide is a polyurethane film.

9. A portable artificial pancreas as defined in any one of claims 1 to 3 wherein the feed pump comprises a rotatable roller holder formed with a plurality of circular bores parallel with the axis of its rotation and equidistantly spaced apart on a circumference centered about the axis of rotation, the roller holder being formed in its outer peripheral side surface with a tube guide groove extending circumferentially thereof and partly positioned in the bores, a plurality of rollers respectively fitted in the bores of the roller holder so as to be rotatable but substantially immovable radially and axially of the holder, and an elastic tube having an intermediate portion reeved around the portions of a predetermined number of the rollers positioned in the gudie groove.

10. A portable artificial pancreas as defined in claim 9 wherein the circular bores extend through the roller holder in the direction of axis of its rotation, and the roller holder is provided on each of its axially opposite end surfaces with a roller retainer for preventing the axial movement of the rollers.

11. A portable artificial pancreas as defined in claim 10 wherein the roller holder is in the form of a short solid cylinder, and the tube guide groove is annular and is formed in the outer peripheral side surface of the roller holder over the entire circumference thereof.

12. A portable artificial pancreas as defined in claim 11 wherein the tube guide groove is substantially semicircular in cross section.

13. A portable artificial pancreas as defined in claim 11 wherein the roller retainer is in the form of a disk concentric with the roller holder.

14. A portable artificial pancreas as defined in claim 8 wherein the roller holder is made of polytetrafluoroethylene, and the rollers are made of stainless steel.

15. A portable artificial pancreas comprising a blood sugar detecting unit including electrode means to be inserted into the living body for measuring the blood sugar of the living body, an injection unit including a container for a blood sugar control agent and a feed pump for the control agent and adapted to inject the control agent into the living body, and an arithmetic control unit including a computer for calculating the blood sugar value from an output signal from the detecting unit and the dose of the control agent based on the value and controlling the pump of the injection unit in accordance with the result of calculation, the artificial pancreas being characterized in that an assembly including the blood sugar detecting unit, an assembly including the injection unit and an assembly including the arithmetic control unit are separate from one another, the assembly including the blood sugar detecting unit and the assembly including the injection unit being individually attachable to the body, the output signal of the detecting unit being adapted to be transmitted to the control unit by radio, the control unit being adapted to transmit a control signal to the injection unit by radio.

16. A portable artificial pancreas as defined in claim 15 wherein the assembly including the blood sugar detecting unit further comprises an arithmetic unit for calculating the blood sugar value from the output signal of the detecting unit and a blood sugar display for showing the result of calculation.

17. A portable artificial pancreas as defined in claim 15 wherein the injection unit comprises a container for a hypoglycemic agent, a container for a hyperglycemic agent, a feed pump for the hypoglycemic agent and a feed pump for the hyperglycemic agent and is adapted to inject the hypoglycemic agent or the hyperglycemic agent into the living body, and the arithmetic control unit calculates the doses of the hypoglycemic agent and the hyperglycemic agent based on the result of calculation of the blood sugar value and controls the pumps of the injection unit in accordance with the result of calculation of the doses.

18. A portable artificial pancreas as defined in any one of claims 15 to 17 wherein the electrode means comprises a bar-like anode insulated except at its front end serving as an electrode reaction portion and at its rear end serving as a lead wire connecting portion, and a cylindrical cathode having the anode fixedly provided therein, and the anode is cover over the surface of its front end with a hydrophilic semipermeable membrane permeable to hydrogen peroxide, then with an immobilized glucose oxidase membrane and further with a hydrophilic semipermeable membrane permeable to glucose, oxygen and hydrogen peroxide by lamination.

19. A portable artificial pancreas as defined in claim 18 wherein the cathode is formed on the outer surface of a cylinder, and the anode is inserted in the cylinder and provided with an insulator fixed at its front end to the front end of the cylinder, the anode and the cathode cylinder being fixed at their rear ends to a plastic plug.

20. A portable artificial pancreas as defined in claim 19 wherein the anode is a noble metal electrode insulated with glass or plastic except at its front end and rear end, and the cathode is a silver electrode or silver chloride electrode formed on the outer surface of a metal cylinder or plastic tube.

21. A portable artificial pancreas as defined in claim 18 wherein the hydrophilic semipermeable membrane permeable to hydrogen peroxide is a cellulose acetate film.

22. A portable artificial pancreas as defined in claim 18 wherein the hydrophilic semipermeable membrane permeable to glucose, oxygen and hydrogen peroxide is a polyurethane film.

23. A portable artificial pancreas as defined in any one of claims 15 to 17 wherein the feed pump comprises a rotatable roller holder formed with a plurality of circular bores parallel with the axis of its rotation and equidistantly spaced apart on a circumference centered about the axis of rotation, the roller holder being formed in its outer peripheral side surface with a tube guide groove extending circumferentially thereof and partly positioned in the bores, a plurality of rollers respectively fitted in the bores of the roller holder so as to be rotatable but substantially immovable radially and axially of the holder, and an elastic tube having an intermediate portion reeved around the portions of a predetermined number of the rollers positioned in the gudie groove.

24. A portable artificial pancreas as defined in claim 23 wherein the circular bores extend through the roller holder in the direction of axis of its rotation, and the roller holder is provided on each of its axially opposite end surfaces with a roller retainer for preventing the axial movement of the rollers.

25. A portable artificial pancreas as defined in claim 24 wherein the roller holder is in the form of a short solid cylinder, and the tube guide groove is annular and is formed in the outer peripheral side surface of the roller holder over the entire circumference thereof.

26. A portable artificial pancreas as defined in claim 25 wherein the tube guide groove is substantially semicircular in cross section.

27. A portable artificial pancreas as defined in claim 25 wherein the roller retainer is in the form of a disk concentric with the roller holder.

28. A portable artificial pancreas as defined in claim 23 wherein the roller holder is made of polytetrafluoroethylene, and the rollers are made of stainless steel.

29. A portable artificial pancreas as defined in claim 15 wherein the single assembly including the arithmetic control unit is provided for a plurality of sets of assemblies including the blood sugar detecting unit and assemblies including the injection unit, and the blood sugar detecting units each have a timer for determining transmitting time and repeatedly transmits an output signal to the control unit in a predetermined order, the control unit comprising a timer for emitting a standard signal corresponding to the transmitting time of each blood sugar detecting unit, a circuit for measuring the time lag between every signal received from each detecting unit and the corresponding standard signal and an alarm for giving warning when the lag exceeds a predetermined value.

0098592

## FIG.1

## FIG. 3

FIG.2

0098592

# FIG.4

1V

voltage

0

T₁ T₂ T₃ T₄    T₅    T₆                    T₇

time

# FIG.5

72 28    62    61
60
68
59
46
70
50

54

57    56
55
53    58

51

52

# FIG.6

49    58

VIII    54    69

57    28

VII    VII

56

71    71
70    70
50    72    62    72
46    VIII    46

0098592

FIG.7

FIG.9

FIG.8

## FIG.10

WAVE FORM SHAPING CIRCUIT (76) → PRESETTABLE SUBSTRACTION COUNTER (73) → MICROCOMPUTER (33)

MOTOR DRIVE CIRCUIT (75) ← MOTOR POWER SUPPLY (74)

FIG.11

# FIG.12

FIG.12 — Block diagram (reference 78): RECEIVER (18) from antenna (17) → MAIN CARRIER DEMODULATOR (19) → FM DEMODULATION CIRCUIT (20) → A-D CONVERTER (32) → MICRO-COMPUTER (33) (block 4). MICRO-COMPUTER connects to BLOOD SUGAR DISPLAY (21), KEYBOARD (22), and D-A CONVERTER (81) → (83)(82) summing → V-F CONVERTER (82) → TRANSMITTER (79) → antenna (80). +1V (110) with switch (111) and +3V (112) with switch (113).

7/10

0098592

## FIG.13

## FIG.14

0098592

## FIG.15

## FIG.16

## FIG.17